(19) Europäisches Patentamt / European Patent Office / Office européen des brevets

(11) **EP 4 583 714 B1**

(12) **EUROPEAN PATENT SPECIFICATION**

(45) Date of publication and mention
of the grant of the patent:
**24.12.2025  Bulletin 2025/52**

(21) Application number: **23767903.0**

(22) Date of filing: **07.09.2023**

(51) International Patent Classification (IPC):
*A23J 3/20* (2006.01)    *A23J 1/00* (2006.01)
*A23J 3/22* (2006.01)

(52) Cooperative Patent Classification (CPC):
**A23J 1/008; A23J 3/20; A23J 3/227**

(86) International application number:
**PCT/EP2023/074670**

(87) International publication number:
**WO 2024/052504 (14.03.2024 Gazette 2024/11)**

(54) **GRANULATE COMPRISIG PARTICLES OF FUNGAL BIOMASS**

GRANULAT ENTHALTEND PARTIKELN AUS PILZBIOMASSE

GRANULAT COMPRENANT DES PARTICULES DE BIOMASSE FONGIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **09.09.2022  EP 22194941**

(43) Date of publication of application:
**16.07.2025  Bulletin 2025/29**

(73) Proprietor: **The Protein Brewery B.V.**
**4817 MV Breda (NL)**

(72) Inventors:
• **KLEINE HAAR, Maloe**
  **4817 MV Breda (NL)**
• **DE LAAT, Wilhelmus Theodorus Antonius Maria**
  **4817 MV Breda (NL)**
• **AKINTOYE, Olumuyiwa Adetokunbo**
  **4817 MV Breda (NL)**

(74) Representative: **Nederlandsch Octrooibureau**
**P.O. Box 29720**
**2502 LS The Hague (NL)**

(56) References cited:
EP-A1- 1 133 926      WO-A1-02/090527
WO-A2-97/36996        FR-A1- 3 022 739
US-A1- 2017 298 318   US-A1- 2020 154 752

## Description

## TECHNICAL FIELD OF THE INVENTION

The present invention relates to a particulate proteinaceous composition comprising at least 80 wt.% of granules having a protein content of at least 30 wt.% and comprising at least 80 wt.% of particles of fungal biomass.

The invention also relates to a process or preparing a particulate proteinaceous composition, said process comprising:

- providing fungal biomass;
- subjecting the fungal biomass to size reduction treatment to produce a powder or a suspension comprising particles of fungal biomass; and
- agglomerating the particles of fungal biomass.

## BACKGROUND OF THE INVENTION

Demand for edible products that can provide a high protein content which is drawn from a non-animal source is increasing. Driven by increasing awareness of personal health, edible products that include non-animal sourced components such as proteins and fibers are considered as a healthier alternative to animal protein based products. In particular, there is growing demand for edible meat substitutes that mimic meat in its composition and texture but are composed of non-animal components, which can reduce reliance animals such as cows, and reduce the carbon footprint posed by such animals.

Single-cell proteins (SCP) are an interesting alternative to meat proteins and as protein source in many other food applications such as breakfast cereals, bread, pasta, dairy, ice cream, chocolate, and soups. When fungal SCP is produced, it can be produced from sugar rich crops in a more sustainable way as compared to the production of meat protein, because SCP yields many more tonnes of protein per hectare compared to meat production and has lower nitrogen emission.

One method for producing a dietary source of protein for human food or animal feed is to produce "single cell protein" (SCP) by means of fermentation (Suman et al., 2015, Int J. Curr. Microbiol. Appl. Sci., Vol 4., No 9., pp 251-262). Fermentation in this respect is understood as the microbial conversion of carbon-rich feedstocks into protein-rich products consisting of microbial cells such as bacteria, yeasts or fungi. The use of SCP as animal feed and food ingredient brings the further advantages that microbial cells have a high content of essential amino acids. Furthermore, in particular fungal cells can be very rich in trace elements and vitamins making the fermented feedstuffs very nutritive.

SCP is already used in food products for human consumption. For instance, Quorn™, comprises a mycoprotein produced as SCP by fermentation of the fungus *Fusarium venenatum.* Quorn™ is available in a variety of forms such as sausages, cutlets, burgers, patties, and strips.

Fermentative production of fungal biomass typically yields a fermentation broth having a dry matter content of around 5 wt.%. This fermentation broth needs to be concentrated and optionally dried in order to render it suitable for shipping and storage under ambient conditions and to make it applicable in a broad range of food products. Concentrated or dried fungal biomass can suitably be applied in food products to provide valuable protein and dietary fibre. However, unlike some other protein sources, such as soy protein isolate, pea protein isolate and gluten, it does not impart significant texture.

US 2008/226811 describes a granulated pea protein composition having a protein content of at least 70 wt.% and a mean diameter of 150 to 300 $\mu$m.

US 2011/311599 describes a granulated powder comprising at least one vegetable protein and at least one vegetable fiber, wherein said granulated powder has:

a laser volume mean diameter $D_{4,3}$ of between 10 $\mu$m and 500 $\mu$m, and
a dry matter content, determined after stoving at 130° C. for 2 hours, of greater than 80%.

WO 2018/029353 describes a process for producing single cell protein (SCP), the process comprising the steps of:

a) growing a thermophilic fungus in a medium containing a fermentable carbon-rich feedstock; wherein the fungus is grown in submerged culture under non-sterile conditions at a temperature higher than 45 °C and a pH of less than 3.8; and,
b) recovery of SCP from the medium in the form of biomass of the thermophilic fungus grown in step a).

US 2020/0093155 describes a method for producing a food ingredient comprising the steps of:

- a. culturing filamentous fungi in growth medium;
- b. harvesting filamentous fungal biomass;
- c. optionally, processing the harvested filamentous fungal biomass;
- d. sizing the biomass to form particles; and
- e. drying the particles to form the food ingredient.

EP 1 133 926 A1 discloses fungal material, e.g. Rhizomucor, consisting of hyphae having a diameter of from 2 to 8 μm. Granules are made by extrusion of of this fungal material. The granules are used as a meat substitute in food compositions such as burgers and sausages.

## SUMMARY OF THE INVENTION

The inventors have unexpectedly discovered that the functionality of proteinaceous fungal biomass can be enhanced by sufficiently reducing the particle size of the fungal biomass and by agglomerating the fine particles of fungal biomass so obtained into granules. These granules typically comprise at 80 wt.% of particles of fungal mass. The protein content of the granules typically is at least 30 wt.%.

Accordingly, the present invention relates to a particulate proteinaceous composition comprising at least 80 wt.% of granules having a protein content of at least 30 wt.% and comprising at least 80 wt.% of particles of fungal biomass, wherein the particle size distribution of the granules meets the following condition:

- 

$$D_{50\text{-grnl}} \geq 50 \ \mu m;$$

$D_{x\text{-grnl}}$ representing the weight percentage of the granules having a diameter of less than x μm;
wherein the particle size distribution of the particles of fungal biomass comprised in the granules meets the following conditions:

- $D_{10\text{-prtcl}} \leq 30 \ \mu m;$
- $2 \ \mu m \leq D_{50\text{-gprtcl}} \leq 60 \ \mu m;$
- $D_{90\text{-prtcl}} \leq 150 \ \mu m;$

  $D_{x\text{-prtcl}}$ representing the weight percentage of the particles of fungal biomass comprised in the granules that have a diameter of less than x μm; and
  wherein the ratio $D_{50\text{-grnl}} : D_{50\text{-prtcl}} \geq 2.0$.

The granules according to the present invention offer the advantage that, in comparison to equally sized non-granulated particles of fungal biomass, they provide better gelling and foaming functionality, as well as a more firm texture when applied in food products, such as meat analogues.

The present invention also provides a process or preparing a particulate proteinaceous composition, said process comprising:

- providing fungal biomass;
- subjecting the fungal biomass to size reduction treatment to produce a powder or a suspension comprising particles of fungal biomass; wherein the particle size distribution of the particles of fungal biomass meets the following condition:

  ○ $D_{10\text{-prtcl}} \leq 30 \ \mu m;$
  ○ $2 \ \mu m \leq D_{50\text{-gprtcl}} \leq 60 \ \mu m;$
  ○ $D_{90\text{-prtcl}} \leq 150 \ \mu m;$

- agglomerating the particles of fungal biomass.

## DETAILED DESCRIPTION OF THE INVENTION

A first aspect of the invention relates to a particulate proteinaceous composition comprising at least 80 wt.% of granules having a protein content of at least 30 wt.% and comprising at least 80 wt.% of particles of fungal biomass, wherein the particle size distribution of the granules meets the following condition:

- 

$$D_{50\text{-}grnl} \geq 50 \text{ μm};$$

$D_{x\text{-}grnl}$ representing the weight percentage of the granules having a diameter of less than x μm;
wherein the particle size distribution of the particles of fungal biomass comprised in the granules meets the following conditions:

- $D_{10\text{-}prtcl} \leq 30$ μm;
- $2$ μm $\leq D_{50\text{-}gprtcl} \leq 60$ μm;
- $D_{90\text{-}prtcl} \leq 150$ μm;

$D_{x\text{-}prtcl}$ representing the weight percentage of the particles of fungal biomass comprised in the granules that have a diameter of less than x μm; and
wherein the ratio $D_{50\text{-}grnl} : D_{50\text{-}prtcl} \geq 2.0$.

The term "fungal biomass" as used herein refers to organic matter that originates from fungi.

The term "protein" as used herein refers to molecules comprising a chain of at least 20 amino acids.

The term "granule" as used herein refers to particle that is an agglomerate of smaller particles, notably of smaller particles of fungal biomass. Granules (or granulates) may be produced by agglomeration techniques such as, for instance, fluid bed agglomeration, compaction, extrusion and spray drying agglomeration.

The term "fat" as used herein refers to lipids containing one or more fatty acid residues. The terms "fat" and "oil" as used herein, unless indicated otherwise, should be considered synonyms.

The term "dietary fibre" as used herein refers to polysaccharides that are not digested by human digestive enzymes. Chitin, chitosan and β-glucans are examples of dietary fibres that are found in fungi.

The term "polysaccharide" as used herein refers to a polymer comprising a long, optionally branched chain of monosaccharides, the total number of monosaccharides in the polysaccharide being at least 10.

The term "a" or "an" as used herein is defined as "at least one" unless specified otherwise.

The term "or" as used herein is defined as "and/or" unless specified otherwise.

Unless indicated otherwise, all percentages mentioned herein should be construed as percentages by weight.

The particle size distributions as referred to herein, unless indicated otherwise, are determined by laser diffraction analysis, using a Mastersizer 3000 (ex Malvern Panalytical). This technique is based on the passing of a laser beam through the dispersed particles. The angular variation in intensity of the scattered light gives information on the particle size. Larger particles scatter light at smaller angles and smaller particles scatter light at larger angles. The scattering intensity data can be used to calculate the particle size distribution.

The particle size distributions of the granules in the particulate proteinaceous composition of the present invention can be determined using a Mastersizer 3000 equipped with an Aero S dry powder dispenser. Also the particle size distribution of the particles of fungal biomass comprised in the granules can suitably be determined using this set-up. The Aero S disperses dry samples by accelerating particles through a venturi using compressed air. The particles are then pulled through the Mastersizer 3000's measurement cell using a vacuum source. The air pressure drop across the venturi is manipulated to achieve complete sample dispersion and can be controlled to within +/-0.1 bar. The particle size distribution of the granules can suitably be determined by the Mastersizer 3000 by employing dispersion pressure of 1 bar in the Aero S dispenser. The particle size distribution of the particles of fungal biomass comprised in the granules can suitably be determined by the Mastersizer 3000 by employing dispersion pressure of 4 bar in the Aero S dispenser. The use the maximum dispersion pressure of 4 bar ensure that the granules are de-agglomerated before the measurement.

The particulate proteinaceous composition of the present invention preferably does not contain any meat or meat derived components. Preferably, the composition is a vegetarian composition, more preferably a vegan composition.

The granules having a protein content of at least 30 wt.% and comprising at least 80 wt.% of particles of fungal biomass preferably constitute at least 90 wt.%, more preferably at least 95 wt.% of the particulate proteinaceous composition of the present invention. Besides these granules, the particulate proteinaceous composition may contain other components such as desiccants, glidants, micronutrients, flavouring and colouring.

According to a particularly preferred embodiment, the particulate proteinaceous composition consists of the granules comprising particles of fungal biomass.

The protein content of the granules preferably is at least 35 wt.%, more preferably 40 to 70 wt.% and most preferably 42 to 60 wt.%.

The particles of biomass in the granules preferably have protein content of at least 35 wt.%, more preferably of 40 to 70 wt.% and most preferably of 42 to 60 wt.%.

Dietary fibre is preferably contained in the particles of fungal biomass in a concentration, calculated by weight of dry matter, of 35-42 wt.%, more preferably of 30-40 wt.%.

Preferably, at least 35 wt.%, more preferably 40-75 wt.% of the dietary fibre in the particles of fungal biomass is aminopolysaccharide selected from chitin, chitosan and combinations. Chitin preferably constitutes 15-50 wt.%, more preferably 20-40 wt.%, most preferably 25-35 wt.% of the dietary fibre in the particles of fungal biomass.

Chitosan preferably constitutes 15-50 wt.%, more preferably 20-40wt.%, most preferably 24-34 wt.% of the dietary fibre in the particles of fungal biomass.

The dietary fibre in the particles of fungal biomass preferably contains 10-70 wt.%, more preferably 20-50 wt.% of polyglucuronic acid.

The combination of the aminopolysaccharide and polyglucuronic acid preferably constitutes at least 50 wt.%, more preferably at least 70 wt.% of the dietary fibre in the particles of fungal biomass.

The combination of fungal protein and the aminopolysaccharide preferably constitutes at least 40 wt.%, more preferably 50-70 wt.% of the dry matter that is contained in the particles of fungal biomass.

The particles of fungal biomass preferably contain, calculated by weight of dry matter, 0-15 wt.%, more preferably 0-10 wt.% and most preferably 0.5-5 wt.% of digestible carbohydrates.

The fat content of the particles of fungal biomass, calculated by weight of dry matter, preferably is in the range of 6-20 wt.%, more preferably of 6.5-12 wt.%.

The combination of protein, fat and dietary fibre preferably constitutes at least 80 wt.%, more preferably 88-99.8 wt.% of the dry matter that is contained in the particles of fungal biomass.

The particulate proteinaceous composition according to the present invention preferably has a water content of 1-15 wt.%, more preferably of 2-10 wt.% and most preferably of 3-9 wt.%.

The granulate of the present invention may suitably be prepared by fluid bed agglomeration, compaction or extrusion. Fluid bed agglomeration typically yields relatively fine granulates, whereas compaction and extrusion are mostly used to produce rather coarse granulates.

Preferably the particle size distribution of the granules is such that $D_{50\text{-grnl}}$ is in the range of 60 to 1,500 $\mu$m, more preferably in the range of 70 to 1,200 $\mu$m and most preferably in the range of 80 to 1,000 $\mu$m.

In a preferred embodiment, the particle size distribution of the granules is such that $D_{10\text{-grnl}}$ is at least 10 $\mu$m, more preferably in the range of 15 to 1,000 $\mu$m and most preferably in the range of 20 to 800 $\mu$m.

Advantageously, the particle size distribution of the granules is such that $D_{90\text{-grnl}}$ is at least 100 $\mu$m, more preferably in the range of 150 to 3,000 $\mu$m and most preferably in the range of 200 to 2,000 $\mu$m.

In a preferred embodiment, the present granulate is a fine granulate having a particle size distribution of the granules that is such that $D_{50\text{-grnl}}$ is in the range of 60 to 500 $\mu$m, more preferably in the range of 70 to 300 $\mu$m and most preferably in the range of 80 to 250 $\mu$m.

In a preferred embodiment, the particle size distribution of the granules of the fine granulate is such that $D_{10\text{-grnl}}$ is at least 10 $\mu$m, more preferably in the range of 15 to 100 $\mu$m and most preferably in the range of 20 to 80 $\mu$m.

Advantageously, the particle size distribution of the granules of the fine granulate is such that $D_{90\text{-grnl}}$ is at least 100 $\mu$m, more preferably in the range of 150 to 1,000 $\mu$m and most preferably in the range of 200 to 800 $\mu$m.

According to a another preferred embodiment, the granules in the particulate composition have a span of less than 8, wherein the span equals $(D_{90}-D_{10})/D_{50}$. More preferably the span of the granules is less than 5, more preferably the span is in the range of 0.8 to 4 and most preferably the span is in the range of 1.5 to 3.

The particles of fungal biomass in the granules of the present invention preferably have a particle size distribution such that $D_{10\text{-prtcl}}$ is not more than 20 $\mu$m, more preferably in the range of 0.5 to 15 $\mu$m and most preferably in the range of 1 to 12 $\mu$m.

Preferably the particle size distribution of the particles of fungal biomass in the granules is such that $D_{50\text{-prtcl}}$ is in the range of 2 to 40 $\mu$m, more preferably in the range of 3 to 30 $\mu$m and most preferably in the range of 4 to 25 $\mu$m.

In a preferred embodiment, the particle size distribution of the particles of fungal biomass is such that $D_{90\text{-prtcl}}$ is in the range of 10 to 130 $\mu$m, more preferably in the range of 20 to 110 $\mu$m and most preferably in the range of 25 to 100 $\mu$m.

According to a particularly preferred embodiment, the granules of the present invention are relatively large agglomerates of the particles of fungal biomass as reflected by a ratio $D_{50\text{-grnl}} : D_{50\text{-prtcl}} \geq 2.0$. Even more preferably, said ratio is at least 3.0. Most preferably, the ratio is in the range of 4.0 to 15.0.

The fungal biomass that is contained in the particles of fungal biomass preferably is biomass of one or more fungi belonging to the class *Zoopagomyceta, Mucoromyceta* or *Symbiomyceta*. Even more preferably, the fungal biomass is biomass of one or more fungi belonging to the phylum *Zoopagomycota* or *Mucoromycota*.

In a preferred embodiment, the fungal biomass in the particles of fungal biomass is biomass of a strain of a fungal genus selected from the group consisting of *Rasamsonia, Talaromyces, Penicillium, Acremonium, Humicola, Paecilo-*

*myces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Myriococcum, Corynascus,* and *Coonemeria,* More preferably, the fungal biomass is biomass of one or more fungi belonging to a species selected from the group consisting of *Rasamsonia composticola Rasamsonia emersonii, Talaromyces emersonii, Rhizomucor miehei, Rhizomucor pusillus, Thermomucor indica-seudaticae, Thielava terricola, Thielava terrestris, Thermoascus thermophilus* and a *Rhizopus* sp., of which the strains *Rasamsonia composticola* strain CBS 141695, *Rasamsonia emersonii* CBS 143030, *Thermomucor indicae-seudaticae* CBS 143027 and CBS 104.75, *Rhizomucor miehei* CBS 143029, *Rhizomucor pusillus* CBS 143028, *Thermoascus thermophilus* CBS 528.71, *Thielavia terrestris* CBS 546.86, *Talaromyces emersonii* CBS 393.64 and *Thermothelomyces thermophila* CBS 117.65 and *Rhizopus* sp. CBS 143160 are more preferred, of which strains CBS 141695, CBS 143030, CBS 143027, CBS 143029, CBS 143160 and CBS 143028 are most preferred.

Another aspect of the invention relates to a process or preparing a particulate proteinaceous composition, preferably a particulate proteinaceous composition according to the present invention, said process comprising:

- providing fungal biomass;
- subjecting the fungal biomass to size reduction treatment to produce a powder or a suspension comprising particles of fungal biomass; wherein the particle size distribution of the particles of fungal biomass meets the following condition:

  ○ $D_{10\text{-prtcl}} \leq 30$ $\mu$m;
  ○ $2$ $\mu$m $\leq D_{50\text{-gprtcl}} \leq 60$ $\mu$m;
  ○ $D_{90\text{-prtcl}} \leq 150$ $\mu$m;

- agglomerating the particles of fungal biomass.

The fungal biomass that is subjected to a size reduction treatment in the present process may be provided in the form of e.g. flocks or a coarse powder. Preferably, the fungal biomass is provided in the form of particulate composition wherein at least 80 wt.% of the particles have a mesh-size of at least 100 $\mu$m, more preferably of a mesh-size of at least 160 $\mu$m and most preferably a mesh-size of at least 200 $\mu$m. The weight percentage of particles having a mesh-size of at least x $\mu$m is determined by employing a mesh with opening of x $\mu$m.

The fungal biomass that is subjected to size reduction treatment preferably comprises at least 10 wt.%, more preferably at least 30 wt.% and most preferably at least 50 wt.% of intact fungal cells, said weight percentage being calculated by weight of the total amount of fungal biomass.

The fungal biomass preferably has a protein content, calculated by weight of dry matter, of at least 30 wt.%, more preferably of at least 35 wt.%, even more preferably of 40 to 70 wt.% and most preferably of 42 to 60 wt.%.

The fungal biomass that is provided in the present process preferably is biomass of one or more fungi belonging to the class *Zoopagomyceta, Mucoromyceta* or *Symbiomyceta.* Even more preferably, the fungal biomass is biomass of one or more fungi belonging to the phylum *Zoopagomycota* or *Mucoromycota.*

In a preferred embodiment, the fungal biomass i is biomass of a strain of a fungal genus selected from the group consisting of *Rasamsonia, Talaromyces, Penicillium, Acremonium, Humicola, Paecilomyces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Myriococcum, Corynascus,* and *Coonemeria,* More preferably, the fungal biomass is biomass of one or more fungi belonging to a species selected from the group consisting of *Rasamsonia composticola Rasamsonia emersonii, Talaromyces emersonii, Rhizomucor miehei, Rhizomucor pusillus, Thermomucor indica-seudaticae, Thielava terricola, Thielava terrestris, Thermoascus thermophilus* and a *Rhizopus* sp., of which the strains *Rasamsonia composticola* strain CBS 141695, *Rasamsonia emersonii* CBS 143030, *Thermomucor indicae-seudaticae* CBS 143027 and CBS 104.75, *Rhizomucor miehei* CBS 143029, *Rhizomucor pusillus* CBS 143028, *Thermoascus thermophilus* CBS 528.71, *Thielavia terrestris* CBS 546.86, *Talaromyces emersonii* CBS 393.64 and *Thermothelomyces thermophila* CBS 117.65 and *Rhizopus* sp. CBS 143160 are more preferred, of which strains CBS 141695, CBS 143030, CBS 143027, CBS 143029, CBS 143160 and CBS 143028 are most preferred.

The size reduction treatment that is used to produce a powder or a suspension comprising particles of fungal biomass preferably comprises milling or grinding.

In an advantageous embodiment of the present process, the size reduction treatment produces a powder comprising the particles of fungal biomass. Such a powder may suitably be agglomerated by means of fluid bed agglomeration, compaction or extrusion.

In another embodiment, the fungal biomass is provided in the form of wet biomass having a water content of 75 to 99 wt.% and the size reduction treatment produces a suspension of the particles of fungal biomass. The particles of fungal biomass in the suspension may suitably be agglomerated by means of, for instance, spray drying agglomeration.

According to a particularly preferred embodiment, the present process yields a particulate proteinaceous composition as defined herein before.

A further aspect of the invention relates to a food product comprising, calculated by weight of dry matter, at least 1 wt.%, preferably 10-95 wt.% of the particulate proteinaceous composition of the present invention.

Examples of food products in which the particulate proteinaceous composition of the present invention may suitably be applied include textured protein (e.g. TVP), meat analogues, meat products, soups, sauces, cereal-based food products and pet food.

Yet another aspect of the invention relates to a method of preparing the aforementioned food product, said method comprising combining the particulate proteinaceous composition of the present invention with one or more other edible ingredients.

The present invention is further illustrated by the following non-limiting examples.

## EXAMPLES

### Example 1: Production of *Rhizomucor pusillus* biomass in cake or powder form

For preculture *Rhizomucor pusillus* strain CBS 143028 was inoculated in 200 ml of a defined mineral medium at pH 5.5 containing KCl 0.17 g/L, $KH_2PO_4$ 1.3 g/L, $Na_2HPO_4$ 0.4 g/L, citric acid 0.5 gr/L, $MgSO_4$.7aq 0.7 gr/L, $FeSO_4$.7aq 0.03 gr/L, $CaCl_2$.2aq 0.035 gr/L, $ZnSO_4$.7aq 0.04 gr/L, $MnCl_2$.4aq 0.004, $CuSO_4$.5aq 0.0005 gr/L, $CoCl_2$.6aq 0.0005 gr/L, $Na2B_4O_7$.10aq 0.003 gr/L, KI 0.0003 gr/L, $Na_2MoO_4$.2aq 0.0005 gr/L, 11 g Dextrose per L; 4 g $(NH_4)_2SO_4$ per L; and 7,5 g tartaric acid per L. The preculture was incubated for 24 hours at 46°C, in a 1 L Erlenmeyer flask with air permeable stop with baffles, in an orbital shaker at 200 rpm. The preculture was then used to inoculate a fermenter containing the defined mineral medium as described above at a pH of 3.5 and comprising 77 g Dextrose per L as C-source; 1.4 g $(NH_4)_2SO_4$ per L as N-source and supplemented with $NH_3$ as titrant. The fungus was grown in the fermenter in fed batch mode at a doubling time of 12 hours. Olive oil was continuously being fed to maintain a concentration of 50 ppm.

Fermentation broths, having reached a dry matter content ranging from 2 to 5 weight percent, were concentrated using a vibrating sieve to achieve a minimum of 10% (w/w) dry matter. The biomass is then mixed with anti-oxidant and pasteurized.

Next the sieved biomass was compressed with a hydraulic press to obtain the *Rhizomucor pusillus* biomass as a cake with a dry matter content of about 29% (w/w). Part of the biomass cake was further freeze dried and milled (6,000 rpm, 0.5 mm mesh size) to obtain the *Rhizomucor pusillus* biomass in powder form with a dry matter content of about 96% (w/w).

The compositions of the *Rhizomucor pusillus* biomass in cake form and powder form were analysed. The results are shown in Tables 1-3.

**Table 1**

| Composition | Biomass in cake form | Biomass in powder form |
|---|---|---|
| Moisture | 71.1 g/100g | 4 g/100g |
| Ash | 1 g/100g | 3.2 g/100g |

**Table 2**

| Nutritional value | *Rhizomucor pusillus* biomass cake (29% dm) | *Rhizomucor pusillus* biomass powder (96% dm) |
|---|---|---|
| Crude protein content* | 14.3 g/100g | 47.6 g/100g |
| Total fat content | 2.4 g/100g | 8.1 g/100g |
| Total dietary fiber** | 10.6 g/100g | 34.8 g/100g |
| Total digestible carbohydrates | 0.8 g/100g | 2.4 g/100g |
| Total sugars | <0.5 g/100g | <0.5 g/100g |
| Energy (kJoules) | 431 kJ/100g | 1429 kJ/100g |
| Energy (kcal) | 103 kcal/100g | 341 kcal/100g |
| * Based on Kjeldahl method (N*6.25). **  Determination of the content of the total number dietary fiber method; enzymatic pretreatment, gravimetr | | |

**Table 3**

| Amino acid profile | *Rhizomucor pusillus* biomass cake (29% dm) |
|---|---|
| Aspartic acid (+ Asparagine) | 1.04 g/100g |
| Threonine* | 0.52 g/100g |
| Serine | 0.52 g/100g |
| Glutamic acid (+ Glutamine ) | 1.30 g/100g |
| Proline | 0.39 g/100g |
| Glycine | 0.48 g/100g |
| Alanine | 0.63 g/100g |
| Citrulline | > 1 g/100g |
| Valine* | 0.61 g/100g |
| Isoleucine* | 0.52 g/100g |
| Leucine* | 0.84 g/100g |
| Tyrosine | 0.42 g/100g |
| Phenylalanine* | 0.47 g/100g |
| Lysine* | 0.80 g/100g |
| Histidine* | 0.27 g/100g |
| Arginine | 0.63 g/100g |
| Cysteine | 0.12 g/100g |
| Methionine* | 0.22 g/100g |
| Tryptophan* | 0.15 g/100g |
| * Essential amino acid for humans. | |

It must be taken into account that the protein content of the *Rhizomucor pusillus* biomass as shown in Table 2 is based on the Kjeldahl method, which is a standard method use to analyze the protein content of different food products. The Kjeldahl method is based on the total nitrogen content and uses a standard conversion factor of 6.25 to estimate the protein content in the analyzed food product. However, a conversion factor of 6.25 may not be appropriate for certain food products.

In the *Rhizomucor pusillus* biomass, the conversion factor may be overestimated due to the presence of other nitrogen sources such as RNA, chitin and chitosan. The real protein content of the *Rhizomucor pusillus* biomass can for this reason better be estimated through amino acid analysis.

**Example 2: Dietary fibre composition of the *Rhizomucor pusillus* biomass**

The dietary fibre content and composition of *the Rhizomucor pusillus* biomass was determined. The results are shown in Tables 4 and 5.

**Table 4**

| Dietary Fiber (DF) content | DF g/100g | Moisture g/100g | % dry matter | DF %dm |
|---|---|---|---|---|
| *Rhizomucor pusillus* biomass (cake) | 10.6 | 71.1 | 29 | 37 |

**Table 5**

| | Batch 1 | Batch 2 | Batch 3 | Batch 4 | Average | Unit |
|---|---|---|---|---|---|---|
| Chitin | 8.6 | 10.8 | 9.8 | 9.9 | 9.8 | g/100 g |
| Chitosan | 9.2 | 9.5 | 8.9 | 9 | 9.2 | g/100 g |

The dry matter-based dietary fiber content of the *Rhizomucor pusillus* biomass is extremely high. Interestingly, the *Rhizomucor pusillus* biomass does not only contain chitin as fiber but contains an almost equal amount of the fiber chitosan. Chitosan is the de-acetylated form of chitin and health benefits have been described for chitosan in animals.

## Example 3: Preparation of a fine powder of *Rhizomucor pusillus* biomass

Milled biomass powders were produced in the same way as described in Example 1 except that next to normally milled biomass powder (Powder 1) also a very finely milled biomass powder (Powder 2) was produced (18,000 rpm, 0.12 mm mesh size). The particle size distributions of the milled powders were determined by means of laser diffraction, using a Mastersizer 3000 equipped with an Aero S dry powder dispenser. The results are summarized in Table 6.

**Table 6**

|  | Powder 1 | Powder 2 |
|---|---|---|
| $D_{10}$ (in $\mu$m) | 6.6 | 2.1 |
| $D_{50}$ (in $\mu$m) | 70 | 11 |
| $D_{90}$ (in $\mu$m) | 240 | 80 |

## Example 4: Rheological test

The milled powders of Example 3 were combined in a ratio of 1:5 (w/w) with 25 mM Tris-HCl buffer pH 8 and stirred for 2.5 hours. Next, the suspensions were centrifuged at 5000 g and separated into a pellet and a supernatant fraction. The supernatants so obtained were each concentrated five times. The concentrated supernatants to obtained had a protein content of about 2.5 mg/mL.

The concentrated supernatants were heated to 80°C for 20 minutes and then cooled down to 25°C. Next the breaking stress and strain of the heated samples was measured with an amplitude sweep measurement using a MCR301 rheometer from Anton Paar.

No breaking stress could be detected in the heated sample that had been produced from the supernatant of the normally milled biomass (Powder 1). The heated sample of the supernatant of the finely milled biomass powder (Powder 2), however, had a breaking stress of 55 Pa, a breaking strain of 1.18% and a storage modulus of 8000 Pa.

## Example 5: Application test

The milled biomass powders of Example 3 were applied in vegan burgers. The compositions of the burgers are shown in Table 7.

**Table 7**

|  | Wt.% | | | |
|---|---|---|---|---|
|  | 1 | 2 | 3 | 4 |
| Powder 1 (hydrated 1:3) | 52 | 54 |  |  |
| Powder 2 (hydrated 1:3) |  |  | 52 | 54 |
| Textured wheat protein [1] (hydrated 1:3) | 44 | 44 | 44 | 44 |
| Egg white | 4 | 2 | 4 | 2 |
| [1] Lory® Tex, ex Loryma GmbH, Germany | | | | |

The burgers were prepared as follows:

- The milled biomass powder and TVP were each hydrated in a volume ratio of 1:3 with tap water
- The hydrated TVP and hydrated biomass powder were mixed together and the egg white was added
- The mixture was mixed for 1 minute with a hand mixer at the lowest setting (1)
- 100 g burgers were formed
- The burgers were left to settle for 10 minutes
- The burgers were steamed for 20 minutes at 100°C

- The burgers were frozen for storage
- The burgers were defrozen on the day of the tasting
- The burgers were baked for 5 minutes at medium/high temperature

The burgers were prepared for consumption and evaluated by an expert panel. The results of the evaluation as summarized in Table 8.

**Table 8**

|  | **Evaluation results** |
| --- | --- |
| Burger 1 | Acceptable texture |
| Burger 2 | Less bite, less firm, more crumbly than Burger 1 |
| Burger 3 | Nice texture. More firm and more dense than Burger 1 |
| Burger 4 | Firmer compared to Burger 2. More moisture than Burger 3 |

### Example 6: Preparation of a granulate of *Rhizomucor pusillus* biomass

Powder 2 of Example 3 is granulated by means of fluid bed agglomeration.

The granulate so obtained has a particle size distribution that meets the specification that is shown in Table 9.

**Table 9**

| $D_{10}$ (in $\mu$m) | 10-50 |
| --- | --- |
| $D_{50}$ (in $\mu$m) | 70-120 |
| $D_{90}$ (in $\mu$m) | 80-500 |

### Example 7: Application test

Powder 2 of Example 3 and the biomass granulate of Example 6 are applied in vegan burgers having the compositions that are shown in Table 10.

**Table 10**

|  | **Wt.%** | |
| --- | --- | --- |
|  | **1** | **2** |
| Powder 2 (hydrated 1:3) | 53 | |
| Biomass granulate (hydrated 1:3) | | 53 |
| Textured wheat protein [1] (hydrated 1:3) | 44 | 44 |
| Egg white | 3 | 3 |
| [1] Lory® Tex, ex Loryma GmbH, Germany | | |

The burgers are prepared for consumption and evaluated by an expert panel. The burgers are found to be very similar and to have a very nice firm texture.

### Example 8: Preparation of a granulate of *Rhizomucor pusillus* biomass

Powder 2 of Example 3 was granulated in a Process 11 twin-screw extruder (Thermo Fischer Scientific, Karlsruhe, Germany). The extrusion conditions employed are summarized in Table 11.

**Table 11**

| During extrusion | Moisture content (%) | 47 |
|---|---|---|
| | Extrusion temperature (°C) | 79 |
| | Cutter speed at end of extruder die (rpm) | 90 |
| After extrusion | Drying temperature (°C) | 70 |
| | Drying time (min) | 60 |

The particle size distribution of the granulate was determined by means of laser diffraction, using a Mastersizer 3000 equipped with an Aero S dry powder dispenser, The so obtained result is shown in Table 12.

**Table 12**

| $D_{10}$ (in $\mu$m) | 513 |
|---|---|
| $D_{50}$ (in $\mu$m) | 899 |
| $D_{90}$ (in $\mu$m) | 1460 |
| Span $(D_{90}-D_{10})/D_{50}$ | 1.06 |

**Claims**

1.  A particulate proteinaceous composition comprising at least 80 wt.% of granules having a protein content of at least 30 wt.% and comprising at least 80 wt.% of particles of fungal biomass, wherein the particle size distribution of the granules meets the following condition:

    • $D_{50\text{-grnl}} \geq 50 \ \mu$m;

    $D_{x\text{-grnl}}$ representing the weight percentage of the granules having a diameter of less than x $\mu$m; wherein the particle size distribution of the particles of fungal biomass comprised in the granules meets the following conditions:

    • $D_{10\text{-prtcl}} \leq 30 \ \mu$m;
    • $2 \ \mu$m $\leq D_{50\text{-gprtcl}} \leq 60 \ \mu$m;
    • $D_{90\text{-prtcl}} \leq 150 \ \mu$m;

    $D_{x\text{-prtcl}}$ representing the weight percentage of the particles of fungal biomass comprised in the granules that have a diameter of less than x $\mu$m; and wherein the ratio $D_{50\text{-grnl}} : D_{50\text{-prtcl}} \geq 2.0$; wherein the particle size distributions are determined using a Mastersizer 3000 equipped with an Aero S dry powder dispenser; and wherein the particle size distribution of the granules is determined by employing a dispersion pressure of 1 bar and wherein the particle size distribution of the particles of fungal biomass comprised in the granules is determined by employing a dispersion pressure of 4 bar.

2.  Particulate proteinaceous composition according to claim 1, wherein the granules meet the following condition:

    • $D_{10\text{-grnl}} \geq 50 \ \mu$m

3.  Particulate proteinaceous composition according to claim 1 or 2, wherein the granules meet the following condition:

    • $D_{90\text{-grnl}} \geq 100 \ \mu$m

4.  Particulate proteinaceous composition according to any one of the preceding claims, wherein composition has a water content of 1-15 wt.%.

5.  Particulate proteinaceous composition according to any one of the preceding claims, wherein the fungal biomass is biomass of one or more fungi belonging to the class *Zoopagomyceta, Mucoromyceta* or *Symbiomyceta.*

6. Particulate proteinaceous composition according to claim 5, wherein the fungal biomass is biomass of one or more fungi belonging to the phylum *Zoopagomycota* or *Mucoromycota.*

7. Particulate proteinaceous composition according to any one of the preceding claims, wherein the fungal biomass is biomass of a strain of a fungal genus selected from the group consisting of *Rasamsonia, Talaromyces, Penicillium, Acremonium, Humicola, Paecilomyces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Myriococcum, Corynascus,* and *Coonemeria.*

8. Particulate proteinaceous composition according to claim 7, wherein the fungal biomass is biomass of a strain of the genus is *Rhizomucor.*

9. A process or preparing a particulate proteinaceous composition according to any one of the preceding claims, said process comprising:

   • providing fungal biomass;
   • subjecting the fungal biomass to size reduction treatment to produce a powder or a suspension comprising particles of fungal biomass; wherein the particle size distribution of the particles of fungal biomass meets the following condition:

   ◦ $D_{10\text{-prtcl}} \leq 30\ \mu m$;
   ◦ $2\ \mu m \leq D_{50\text{-gprtcl}} \leq 60\ \mu m$;
   ◦ $D_{90\text{-prtcl}} \leq 150\ \mu m$;

   • agglomerating the particles of fungal biomass;

   wherein the particle size distributions are determined using a Mastersizer 3000 equipped with an Aero S dry powder dispenser; and wherein the particle size distribution of the granules is determined by employing a dispersion pressure of 1 bar and wherein the particle size distribution of the particles of fungal biomass comprised in the granules is determined by employing a dispersion pressure of 4 bar.

10. Process according to any one of claims 9, wherein the fungal biomass is provided in the form of a particulate composition having a $D_{50}$ of at least 80 $\mu m$ or in the form of a suspension comprising suspended material having a $D_{50}$ of at least 80 $\mu m$.

11. Process according to any one of claims 9 or 10, wherein the size reduction treatment comprises milling or grinding.

12. Process according to any one of claims 9-11, wherein the size reduction treatment produces a powder comprising the particles of fungal biomass.

13. Process according to any one of claims 9-12, wherein the particles of fungal biomass are agglomerated by means of fluid bed granulation, compaction, extrusion or spray drying agglomeration.

14. A food product comprising, calculated by weight of dry matter, at least 1 wt.%, preferably 10-95 wt.% of the particulate proteinaceous composition according to any one of claims 1-8.

15. A method of preparing a food product according to claim 14, said method comprising combining the particulate proteinaceous composition according to any one of claims 1-8 with one or more other edible ingredients.


**Patentansprüche**

1. Partikuläre proteinhaltige Zusammensetzung die mindestens 80 Gew.-% Granulatkörner enthalt, die einen Proteingehalt von mindestens 30 Gew.-% aufweisen und die mindestens 80 Gew.-% Teilchen aus Pilzbiomasse enthalt, wobei die Teilchengrößenverteilung der Granulatkörner die folgende Bedingung erfüllt:

- $D_{50\text{-}grnl} \geq 50\ \mu m$;

wobei $D_{x\text{-}grnl}$ den Massenanteil der Granulatkörner, die einen Durchmesser von weniger als $x\ \mu m$ aufweisen, darstellt;

wobei die Teilchengrößenverteilung der in den Granulatkörnern enthaltenen Teilchen aus Pilzbiomasse die folgenden Bedingungen erfüllt:

- $D_{10\text{-}prtcl} \leq 30\ \mu m$;
- $2\ \mu m \leq D_{50\text{-}gprtcl} \leq 60\ \mu m$;
- $D_{90\text{-}prtcl} \leq 150\ \mu m$;

wobei $D_{x\text{-}prtcl}$ den Massenanteil der in den Granulatkörnern umfassten Teilchen aus Pilzbiomasse darstellt, die einen Durchmesser von weniger als $x\ \mu m$ aufweisen; und

wobei das Verhältnis $D_{50\text{-}grnl} : D_{50\text{-}prtcl} \geq 2{,}0$;

wobei die Teilchengrößenverteilungen unter Verwendung eines mit einer Aero S-Trockendispergiereinheit ausgestatteten Mastersizer 3000 bestimmt werden; und wobei die Teilchengrößenverteilung der Granulatkörner unter Verwendung eines Dispersionsdrucks von 1 bar bestimmt wird und wobei die Teilchengrößenverteilung der in den Granulatkörnern umfassten Teilchen aus Pilzbiomasse unter Verwendung eines Dispersionsdrucks von 4 bar bestimmt wird.

2. Partikuläre proteinhaltige Zusammensetzung nach Anspruch 1, wobei die Granulatkörner die folgende Bedingung erfüllen:

- $D_{10\text{-}grnl} \geq 50\ \mu m$

3. Partikuläre proteinhaltige Zusammensetzung nach Anspruch 1 oder 2, wobei die Granulatkörner die folgende Bedingung erfüllt:

- $D_{90\text{-}grnl} \geq 100\ \mu m$

4. Partikuläre proteinhaltige Zusammensetzung nach einem der vorstehenden Ansprüche, wobei die Zusammensetzung einen Wassergehalt von 1-15 Gew.-% aufweist.

5. Partikuläre proteinhaltige Zusammensetzung nach einem der vorstehenden Ansprüche, wobei die Pilzbiomasse Biomasse eines oder mehrerer Pilze ist, die der Klasse *Zoopagomyceta, Mucoromyceta* oder *Symbiomyceta* angehören.

6. Partikuläre proteinhaltige Zusammensetzung nach Anspruch 5, wobei die Pilzbiomasse Biomasse eines oder mehrerer Pilze ist, die dem Phylum *Zoopagomyceta* oder *Mucoromyceta* angehören.

7. Partikuläre proteinhaltige Zusammensetzung nach einem der vorstehenden Ansprüche, wobei die Pilzbiomasse Biomasse eines Stammes einer Pilzgattung ausgewählt aus der Gruppe bestehend aus *Rasamsonia, Talaromyces, Penicillium, Acremonium, Humicola, Paecilomyces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Myriococcum, Corynascus,* und *Coonemeria* ist.

8. Partikuläre proteinhaltige Zusammensetzung nach Anspruch 7, wobei die Pilzbiomasse Biomasse eines Stammes der Gattung *Rhizomucor* ist.

9. Verfahren zum Herstellen einer partikulären proteinhaltigen Zusammensetzung nach einem der vorstehenden Ansprüche, besagtes Verfahren umfassend:

- Bereitstellen von Pilzbiomasse;
- Unterziehen der Pilzbiomasse einer Größenreduktionsbehandlung, um ein Pulver oder eine Aufschlämmung herzustellen, das/die Teilchen aus Pilzbiomasse enthält; wobei die Teilchengrößenverteilung der Teilchen aus Pilzbiomasse die folgende Bedingung erfüllt:

  ○ $D_{10\text{-}prtcl} \leq 30\ \mu m$;

◦ $2\ \mu m \le D_{50\text{-gprtcl}} \le 60\ \mu m$;
◦ $D_{90\text{-prtcl}} \le 150\ \mu m$;

• Agglomerieren der Teilchen aus Pilzbiomasse;

wobei die Teilchengrößenverteilungen unter Verwendung eines mit einer Aero S-Trockendispergiereinheit ausgestatteten Mastersizer 3000 bestimmt werden; und wobei die Teilchengrößenverteilung der Granulatkörner unter Verwendung eines Dispersionsdrucks von 1 bar bestimmt wird und wobei die Teilchengrößenverteilung der in den Granulatkörnern umfassten Teilchen aus Pilzbiomasse unter Verwendung eines Dispersionsdrucks von 4 bar bestimmt wird.

10. Verfahren nach einem der Ansprüche 9, wobei die Pilzbiomasse in der Form einer partikulären Zusammensetzung, die einem $D_{50}$ von mindestens 80 $\mu m$ aufweist, oder in der Form einer Aufschlämmung enthaltend aufgeschlämmtes Material, das einem $D_{50}$ von mindestens 80 $\mu m$ aufweist, bereitgestellt wird.

11. Verfahren nach einem der Ansprüche 9 oder 10, wobei die Größenreduktionsbehandlung Mahlen oder Zerquetschen umfasst.

12. Verfahren nach einem der Ansprüche 9-11, wobei die Größenreduktionsbehandlung ein die Teilchen aus Pilzbiomasse enthaltendes Pulver herstellt.

13. Verfahren nach einem der Ansprüche 9-12, wobei die Teilchen aus Pilzbiomasse mittels Fließbettgranulierung, Verdichtung, Extrusion oder Sprühtrocknungsagglomeration agglomeriert werden.

14. Lebensmittelprodukt enthaltend, berechnet nach Gewicht der Trockenmasse, mindestens 1 Gew.-%, bevorzugt 10-95 Gew.-% der partikulären proteinhaltigen Zusammensetzung nach einem der Ansprüche 1- 8 enthält.

15. Methode zum Herstellen eines Lebensmittelprodukts nach Anspruch 14, wobei besagtes Verfahren das Kombinieren der partikulären proteinhaltigen Zusammensetzung nach einem der Ansprüche 1-8 mit einem oder mehreren anderen essbaren Bestandteilen umfasst.


**Revendications**

1. Composition protéique particulaire comprenant au moins 80 % en poids de granulés ayant une teneur en protéines d'au moins 30 % en poids et comprenant au moins 80 % en poids de particules de biomasse fongique, où la distribution granulométrique des granulés répond à la condition suivante :

• $D_{50\text{-grnl}} \ge 50\ \mu m$ ;

$D_{x\text{-grnl}}$ représentant le pourcentage en poids des granulés ayant un diamètre inférieur à x $\mu m$ ;
où la distribution granulométrique des particules de biomasse fongique comprises dans les granulés répond aux conditions suivantes :

• $D_{10\text{-prtcl}} \le 30\ \mu m$ ;
• $2\ \mu m \le D_{50\text{-gprtcl}} \le 60\ \mu m$ ;
• $D_{90\text{-prtcl}} \le 150\ \mu m$ ;

$D_{x\text{-prtcl}}$ représentant le pourcentage en poids des particules de biomasse fongique comprises dans les granulés qui ont un diamètre inférieur à x $\mu m$ ; et
où le rapport $D_{50\text{-grnl}} : D_{50\text{-prtcl}} \ge 2.0$ ;
où les distributions granulométriques sont déterminées à l'aide d'un Mastersizer 3000 équipé d'un disperseur de poudre sèche Aero S ; et où la distribution granulométrique des granulés est déterminée en utilisant une pression de dispersion de 1 bar et où la distribution granulométrique des particules de biomasse fongique comprises dans les granulés est déterminée en utilisant une pression de dispersion de 4 bars.

2. Composition protéique particulaire selon la revendication 1, où les granulés répondent à la condition suivante :

• $D_{10\text{-grnl}} \geq 50\ \mu m$

3. Composition protéique particulaire selon la revendication 1 ou 2, où les granulés répondent à la condition suivante :

• $D_{90\text{-grnl}} \geq 100\ \mu m$

4. Composition protéique particulaire selon l'une quelconque des revendications précédentes, où la composition a une teneur en eau de 1 à 15 % en poids.

5. Composition protéique particulaire selon l'une quelconque des revendications précédentes, où la biomasse fongique est une biomasse d'un ou plusieurs champignons appartenant à la classe des *Zoopagomyceta, Mucoromyceta* ou *Symbiomyceta.*

6. Composition protéique particulaire selon la revendication 5, où la biomasse fongique est une biomasse d'un ou plusieurs champignons appartenant au phylum *Zoopagomycota* ou *Mucoromycota.*

7. Composition protéique particulaire selon l'une quelconque des revendications précédentes, où la biomasse fongique est une biomasse d'une souche d'un genre fongique choisi dans le groupe constitué par *Rasamsonia, Talaromyces, Penicillium, Acremonium, Humicola, Paecilomyces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Myriococcum, Corynascus* et *Coonemeria.*

8. Composition protéique particulaire selon la revendication 7, où la biomasse fongique est une biomasse d'une souche du genre *Rhizomucor.*

9. Processus ou préparation d'une composition protéique particulaire selon l'une quelconque des revendications précédentes, ledit processus comprenant :

• fournir une biomasse fongique ;
• soumettre la biomasse fongique à un traitement de réduction de taille pour produire une poudre ou une suspension comprenant des particules de biomasse fongique ; où la distribution granulométrique des particules de biomasse fongique répond à la condition suivante :

◦ $D_{10\text{-prtcl}} \leq 30\ \mu m$ ;
◦ $2\ \mu m \leq D_{50\text{-gprtcl}} \leq 60\ \mu m$ ;
◦ $D_{90\text{-prtcl}} \leq 150\ \mu m$ ;

• agglomérer les particules de biomasse fongique ;

où les distributions granulométriques sont déterminées à l'aide d'un Mastersizer 3000 équipé d'un disperseur de poudre sèche Aero S ; et où la distribution granulométrique des granulés est déterminée en utilisant une pression de dispersion de 1 bar et où la distribution granulométrique des particules de biomasse fongique comprises dans les granulés est déterminée en utilisant une pression de dispersion de 4 bars.

10. Processus selon l'une quelconque des revendications 9, où la biomasse fongique est fournie sous la forme d'une composition particulaire ayant un $D_{50}$ d'au moins 80 $\mu m$ ou sous la forme d'une suspension comprenant un matériau en suspension ayant un $D_{50}$ d'au moins 80 $\mu m$.

11. Processus selon l'une quelconque des revendications 9 ou 10, où le traitement de réduction de taille comprend un broyage ou un concassage.

12. Processus selon l'une quelconque des revendications 9 à 11, où le traitement de réduction de taille produit une poudre comprenant les particules de biomasse fongique.

13. Processus selon l'une quelconque des revendications 9 à 12, où les particules de biomasse fongique sont agglomérées au moyen d'une granulation en lit fluidisé, d'un compactage, d'une extrusion ou d'une agglomération par séchage par atomisation.

**14.** Produit alimentaire comprenant, calculé en poids de matière sèche, au moins 1 % en poids, de préférence 10 à 95 % en poids, de la composition protéique particulaire selon l'une quelconque des revendications 1 à 8.

**15.** Procédé de préparation d'un produit alimentaire selon la revendication 14, ledit procédé comprenant la combinaison de la composition protéique particulaire selon l'une quelconque des revendications 1 à 8 avec un ou plusieurs autres ingrédients comestibles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2008226811 A
- US 2011311599 A
- WO 2018029353 A
- US 20200093155 A
- EP 1133926 A1

### Non-patent literature cited in the description

- **SUMAN et al.** *Int J. Curr. Microbiol. Appl. Sci.*, 2015, vol. 4 (9), 251-262